# EUROPEAN PATENT SPECIFICATION

(11) **EP 4 162 876 B1**
(45) Date of publication and mention of the grant of the patent: **07.10.2026**
(21) Application number: 21843305.0
(22) Date of filing: 07.07.2021
(51) Int. Cl.: A61B 5/327, A61B 5/349, A61B 5/00, G16H 50/20, G06N 3/08

(54) **ELECTROCARDIOGRAM GENERATION DEVICE BASED ON GENERATIVE ADVERSARIAL NETWORK ALGORITHM, AND METHOD THEREOF**
ELEKTROKARDIOGRAMMERZEUGUNGSVORRICHTUNG AUF DER BASIS EINES GENERATIVEN KONTRADIKTORISCHEN NETZWERKALGORITHMUS UND VERFAHREN DAFÜR
DISPOSITIF DE GÉNÉRATION D'ÉLECTROCARDIOGRAMME BASÉ SUR UN ALGORITHME DE RÉSEAU ANTAGONISTE GÉNÉRATIF, ET PROCÉDÉ ASSOCIÉ

(30) Priority: 14.07.2020 KR 20200086485
(43) Date of publication of application: 12.04.2023
(73) Proprietor: Bodyfriend Co., Ltd., Seoul 06302 (KR); Medical AI, Seoul 04194 (KR)
(72) Inventor: KWON, Joon Myoung, Seoul 06302 (KR)
(74) Representative: Isarpatent
(86) International application number: PCT/KR2021/008627
(87) International publication number: WO 2022/014943

(56) References cited:
- JP-A- 2005 323 821
- JP-A- 2020 042 598
- KR-A- 20190 114 694
- KR-B1- 102 078 703
- LEE JEEEUN ET AL: "Synthesis of Electrocardiogram V-Lead Signals From Limb-Lead Measurement Using R-Peak Aligned Generative Adversarial Network", IEEE JOURNAL OF BIOMEDICAL AND HEALTH INFORMATICS, IEEE, PISCATAWAY, NJ, USA, vol. 24, no. 5, 21 August 2019 (2019-08-21), pages 1265 - 1275, XP011786355, ISSN: 2168-2194, [retrieved on 20200504], DOI: 10.1109/JBHI.2019.2936583
- TOMER GOLANY ET AL: "SimGANs: Simulator-Based Generative Adversarial Networks for ECG Synthesis to Improve Deep ECG Classification", ARXIV.ORG, CORNELL UNIVERSITY LIBRARY, 201 OLIN LIBRARY CORNELL UNIVERSITY ITHACA, NY 14853, 27 June 2020 (2020-06-27), XP081707912
- KIM, MIN-GU; PAN, SUNG BUM: "A Sudy on Simulated ECG Generation of GAN based using Axiliary Cassifier", PROCEEDINGS OF KIIT CONFERENCE, 1 January 2019 (2019-01-01), Korea, pages 120 - 121, XP009533428

## Description

### Technical Field

The present invention relates to an apparatus and method for generating an electrocardiogram based on a generative adversarial network algorithm, and more particularly to an apparatus and method for generating an electrocardiogram based on a generative adversarial network algorithm that generate information based on 12-n electrodes from information based on n electrodes by using a deep learning algorithm and predict a patient's condition using the generated information based on the electrodes.

### Background Art

An electrocardiogram is a graphic record of potentials related to heartbeat on the surface of the body. In addition to standard 12-lead electrocardiograms, there are exercise load electrocardiograms and electrocardiograms during activity (Holter monitor and event monitor electrocardiograms). Although many tests are used for diagnosing circulatory diseases, electrocardiography has many advantages and is the most commonly used test in clinical practice among the above tests. Electrocardiography is a non-invasive test that is accurate, simple, reproducible, easily repeatable, and inexpensive. Electrocardiography is the most widely used for the diagnosis of arrhythmias and coronary artery disease.

In a standard 12-lead electrocardiogram, six electrodes are attached to the front of the chest and three electrodes are attached to the limbs, and then all pieces of 12-lead information are collected and combined to be used to diagnose a disease. However, since 12 lead electrodes cause the chest to be exposed and it is difficult to attach all 12 electrodes, it is difficult to measure a standard 12-lead electrocardiogram at home or in daily life.

Recently, wearable electrocardiogram devices that measure an electrocardiogram using only six-electrode information by means of three of the 12 electrodes on the limbs or using only one-electrode information, such as a patch-type product, are being developed.

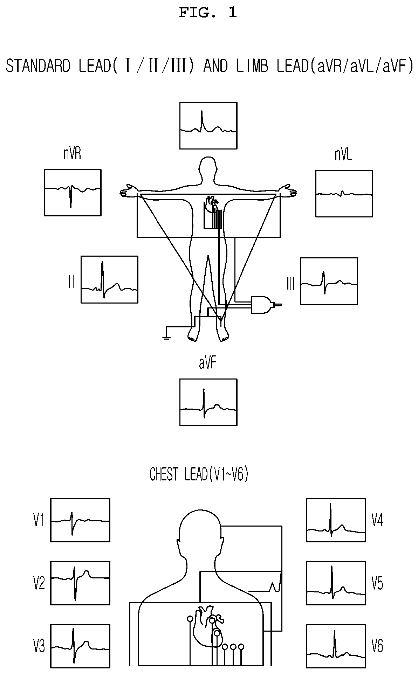

FIG. 1 is a diagram showing 12-lead electrocardiogram data.

For example, as shown in FIG. 1, an electrocardiogram based on 12 lead electrodes used in a hospital is measured by measuring the electrocardiogram data of a total of 12 leads in the left and right boxes simultaneously. However, a wearable electrocardiogram device uses only the leftmost information based on limb electrodes (measures information based on six electrodes I, II, III, aVL, aVF, and aVL by attaching three electrodes to the limbs), or collects only electrocardiogram information based on one, such as I or II, of the electrodes.

When only information based on six electrodes or only information based on one electrode is used as described above, there is a problem in that accuracy is lowered because only half or 1/12 of original information based on 12 lead electrodes is used.

The technology that is the background of the present invention is disclosed in Korean Patent No. 10-1109738 (published on February 24, 2012).

Meanwhile, prior art 1 (LEE JEEEUN ET AL: "Synthesis of Electrocardiogram V-Lead Signals From Limb-Lead Measurement Using R-Peak Aligned Generative Adversarial Network") performs R-peak alignment to preserve the physiological information of the ECG, and converts the time-domain ECG to two-dimensional space through ordered time-sequence embedding. Finally, the GAN is trained on the pairs between the modified limb II (MLII) leads and each thoracic (V) lead.

Prior art 2 (TOMER GOLANY ET AL: "SimGANs: Simulator-Based Generative Adversarial Networks for ECG Synthesis to Improve Deep ECG Classification") uses a system of ordinary differential equations representing cardiac mechanics and incorporates this ODE system into the optimization process of a generative adversarial network to generate biologically plausible ECG training examples.

### Disclosure

### Technical Problem

An object of the present invention is to provide an apparatus and method for generating an electrocardiogram based on a generative adversarial network algorithm that generate information based on 12-n electrodes from information based on n electrodes by using a deep learning algorithm and predict a patient's condition using the generated information based on the electrodes.

### Technical Solution

In order to accomplish the above object, an apparatus for generating an electrocardiogram based on a deep learning algorithm according to the present invention is provided, as defined by independent claim 1. The apparatus includes: an input unit configured to receive the electrocardiogram data of a patient who wants his or her disease to be diagnosed; a data generation unit configured to generate a plurality of synthesized electrocardiogram data by inputting the received electrocardiogram data to a previously trained generative adversarial network algorithm; and an output unit configured to output the received actual electrocardiogram data of the patient, the plurality of generated electrocardiogram data, and a diagnosis result.

According to the present invention, the apparatus further includes a training unit configured to extract lead electrocardiogram data from overall electrocardiogram data of a patient diagnosed with a heart disease and to train to generate a plurality of synthesized electrocardiogram data by inputting the extracted lead electrocardiogram data to a previously constructed generative adversarial network algorithm.

According to the present invention, the training unit includes: a first generative model configured to generate n pieces of synthesized electrocardiogram data from the lead electrocardiogram data extracted from the input overall electrocardiogram data; and a second generative model configured to generate m pieces of synthesized electrocardiogram data from the n pieces of synthesized electrocardiogram data generated by the first generative model.

The training unit may include: a first discriminative model configured to receive the lead electrocardiogram data or the m pieces of synthesized electrocardiogram data and to determine whether the data is actual data or has been synthesized; and a second discriminative model configured to receive overall electrocardiogram data exclusive of the lead electrocardiogram data or n pieces of synthesized electrocardiogram data and to determine whether the data is actual data or has been synthesized.

Furthermore, according to the present invention, a method of generating an electrocardiogram based on a deep learning algorithm by using an apparatus for generating an electrocardiogram is provided, as defined by independent claim 3. The method includes: extracting lead electrocardiogram data from overall electrocardiogram data of a patient diagnosed with a heart disease and training to generate a plurality of synthesized electrocardiogram data by inputting the extracted lead electrocardiogram data to a previously constructed generative adversarial network algorithm; receiving the electrocardiogram data of a patient who wants his or her disease to be diagnosed; generating a plurality of synthesized electrocardiogram data by inputting the received electrocardiogram data to a previously trained generative adversarial network algorithm; and outputting the received actual electrocardiogram data of the patient, the plurality of generated electrocardiogram data, and a diagnosis result.

The step of training to generate the plurality of synthesized electrocardiogram data comprises: generating n pieces of synthesized electrocardiogram data from the lead electrocardiogram data extracted from the input overall electrocardiogram data by using a first generative model; and generating m pieces of synthesized electrocardiogram data from the n pieces of synthesized electrocardiogram data generated by the first generative model by using a second generative model.

### Advantageous Effects

As described above, according to the present invention, n pieces of additional electrocardiogram data are generated using electrocardiogram data, measured from one electrode or three electrodes, by means of a deep learning algorithm, thereby increasing the accuracy of diagnosing heart-related diseases. Furthermore, according to the present invention, it may be applied to a portable wearable electrocardiogram device, so that it can be used at home or in daily life.

### Description of Drawings

FIG. 1 is a diagram illustrating a general standard 12-lead electrocardiography method;
FIG. 2 is a block diagram showing an apparatus for generating an electrocardiogram based on a generative adversarial network algorithm according to an embodiment of the present invention;
FIG. 3 is a flowchart showing the operation flow of a method of generating an electrocardiogram based on a generative adversarial network algorithm according to an embodiment of the present invention;
FIG. 4 is a view showing the types of electrocardiogram data; and
FIG. 5 is a view illustrating generative models and discriminative models according to an embodiment of the present invention.

### Best Mode

In an apparatus for generating an electrocardiogram based on a deep learning algorithm, the apparatus includes: an input unit configured to receive the electrocardiogram data of a patient who wants his or her disease to be diagnosed; a data generation unit configured to generate a plurality of synthesized electrocardiogram data by inputting the received electrocardiogram data to a previously trained generative adversarial network algorithm; and an output unit configured to output the received actual electrocardiogram data of the patient, the plurality of generated electrocardiogram data, and a diagnosis result.

### Mode for Invention

Preferred embodiments according to the present invention will be described in detail with reference to the accompanying drawings below. In this process, the thicknesses of the lines or the sizes of the components shown in the drawings may be exaggerated for the clarity and convenience of description.

Furthermore, the terms to be described later are terms defined by taking into consideration functions in the present invention, and may vary according to the intention or custom of a user or operator. Therefore, definitions of these terms should be made based on the content throughout this specification.

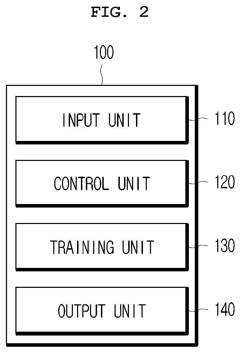

First, an apparatus for generating an electrocardiogram based on a deep learning algorithm according to an embodiment of the present invention will be described with reference to FIG. 2.

FIG. 2 is a block diagram showing an apparatus for generating an electrocardiogram based on a generative adversarial network algorithm according to an embodiment of the present invention.

As shown in FIG. 2, the apparatus 100 for generating an electrocardiogram based on a generative adversarial network algorithm according to the embodiment of the present invention includes an input unit 110, a data generation unit 120, a training unit 130, and an output unit 140.

First, the input unit 110 receives the electrocardiogram data of a patient. In this case, the input unit 110 additionally receives an indicator indicating which signals the received electrocardiogram data are.

The data generation unit 120 generates a plurality of synthesized electrocardiogram data by inputting the received electrocardiogram data to a generative adversarial network algorithm that has been trained.

The training unit 130 receives electrocardiogram data measured from a patient having a heart disease. Furthermore, lead data is extracted from the received electrocardiogram data, and training is performed to output synthesized electrocardiogram data by inputting the extracted lead data to the generative adversarial network algorithm.

In this case, the training unit 130 constructs pluralities of generative models and discriminative models based on the generative adversarial network algorithm. Accordingly, the training unit 130 trains the generative models to generate the synthesized electrocardiogram data, and trains the discriminative models to determine whether data has been synthesized by analyzing the generated synthesized electrocardiogram data and actual electrocardiogram data.

Finally, the output unit outputs a diagnosis result using a plurality of electrocardiogram data generated by synthesis with the received electrocardiogram data.

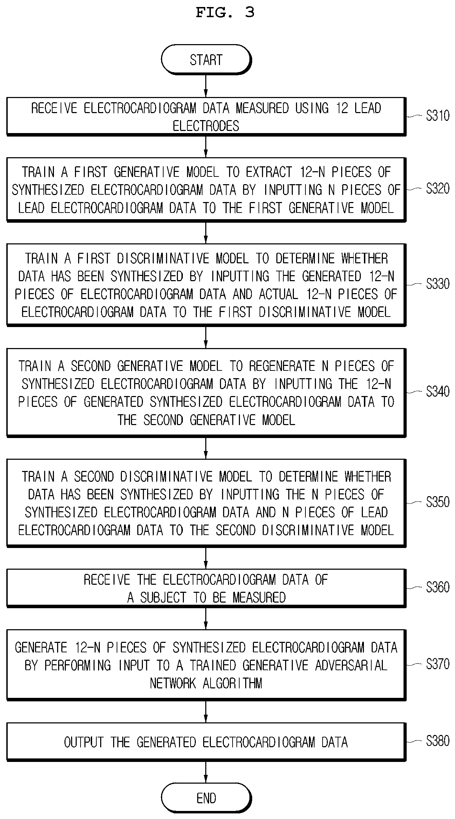

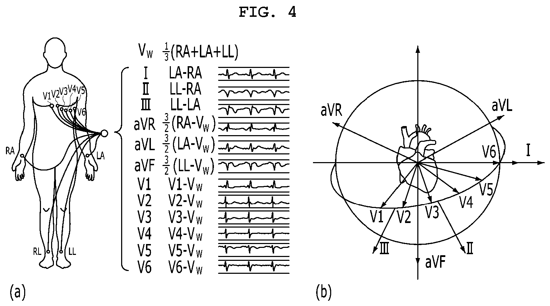

A method of generating electrocardiogram data will be described in more detail below by using FIGS. 3 and 4.

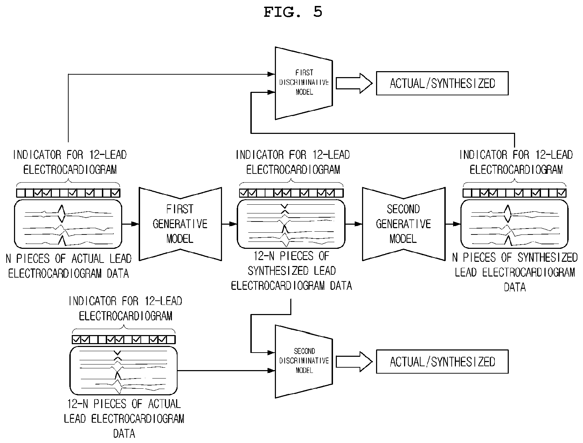

FIG. 3 is a flowchart showing the operation flow of a method of generating an electrocardiogram based on a generative adversarial network algorithm according to an embodiment of the present invention, FIG. 4 is a view showing the types of electrocardiogram data, and FIG. 5 is a view illustrating generative models and discriminative models according to an embodiment of the present invention.

As shown in FIG. 3, the input unit 110 according to the embodiment of the present invention receives electrocardiogram data measured using 12 lead electrodes in step S310.

In other words, the input unit 110 receives electrocardiogram data measured in such a manner that 12 lead electrodes are attached to a body part of a patient diagnosed with arrhythmia or a heart disease. In this case, the input unit 110 additionally receives an indicator indicating which signals the received electrocardiogram data are.

Although the 12 lead electrodes are described for the received electrocardiogram data in the embodiment of the present invention, the present invention is not limited thereto. If necessary, electrocardiogram data obtained from 6 lead electrodes, 18 lead electrodes, or 24 lead electrodes may be used.

Thereafter, the input unit 110 transfers received 12 pieces of electrocardiogram data and corresponding diagnosis results to the training unit 130.

Then, the training unit 130 extracts n pieces of lead electrocardiogram data from the 12 pieces of electrocardiogram data, and trains a first generative model to extract 12-n pieces of synthesized electrocardiogram data by inputting the extracted n pieces of lead electrocardiogram data to the first generative model in step S320.

In other words, the training unit 130 collects electrocardiogram data measured from a plurality of patients. Thereafter, the training unit 130 extracts n pieces of lead electrocardiogram data from each group. The extracted lead electrocardiogram data is input to the first generative model based on the generative adversarial network algorithm.

In other words, as shown in FIG. 4, an electrocardiogram is recorded as a graph including three standard leads I, II, and III, three limb leads aVR, aVR, and aVF, and six chest leads V1 to V6.

Accordingly, the first generative model receives n pieces of lead electrocardiogram data and an indicator for signals corresponding to the lead electrocardiogram data.

Then, the first generative model generates the remaining pieces of electrocardiogram data by synthesizing the remaining pieces of electrocardiogram data except for the received lead electrocardiogram data.

For example, assuming that the received lead electrocardiogram data is three pieces of limb lead data, the first generative model generates nine pieces of synthesized electrocardiogram data exclusive of the three pieces of limb lead data.

Thereafter, the training unit 130 trains a first discriminative model to determine whether data has been synthesized by inputting the 12-n pieces of electrocardiogram data generated through the synthesis and actual 12-n pieces of electrocardiogram data to the first discriminative model in step S330.

In other words, the first discriminative model mutually analyzes nine pieces of synthesized electrocardiogram data and nine pieces of actual electrocardiogram data exclusive of three pieces of limb lead data. In addition, the first discriminative model determines whether the nine pieces of synthesized electrocardiogram data are synthesized electrocardiogram data or actual electrocardiogram data.

Thereafter, the training unit 130 trains a second generative model to regenerate n pieces of synthesized electrocardiogram data by inputting the 12-n pieces of synthesized electrocardiogram data, generated in step S320, to the second generative model in step S340.

In step S320, the first generative model generates nine pieces of synthesized electrocardiogram data from three pieces of limb lead data. Accordingly, the second generative model generates three pieces of limb lead data by synthesizing the three pieces of limb lead data using the nine pieces of synthesized electrocardiogram data.

When step S340 is completed, the training unit 130 trains a second discriminative model to determine whether data has been synthesized by inputting the n pieces of synthesized electrocardiogram data and n pieces of lead electrocardiogram data to the second discriminative model in step S350.

The training unit 130 repeatedly trains the generative models for generating electrocardiogram data and the discriminative models for determining whether data has been synthesized to generate an electrocardiogram model close to a real one.

Meanwhile, although the two generative models and the two discriminative models are constructed such that they can compete with each other in the embodiment of the present invention, as shown in FIG. 5, the numbers of generative models and discriminative models to be constructed may be increased or decreased.

When step S350 is completed, the input unit 110 receives the electrocardiogram data of a subject to be measured in step S360.

In this case, the received electrocardiogram data is part of electrocardiogram data rather than all 12 pieces of electrocardiogram data. The input unit 110 transfers the electrocardiogram data to the control unit 120.

Then, the control unit 120 generates 12-n pieces of synthesized electrocardiogram data by inputting the received n pieces of electrocardiogram data as lead electrocardiogram data to the generative adversarial network algorithm that has been trained in step S370.

In the case of a wearable device, an electrocardiogram is measured using one to three electrodes. Accordingly, the control unit 120 inputs one or three pieces of electrocardiogram data to the generative adversarial network algorithm.

Then, the generative adversarial network algorithm generates 11 pieces of synthesized electrocardiogram data from one piece of lead electrocardiogram data. Alternatively, when receiving three pieces of lead electrocardiogram data, the generative adversarial network algorithm generates nine pieces of synthesized electrocardiogram data.

Thereafter, the output unit 140 outputs the generated electrocardiogram data in step S380.

In other words, the output unit 140 outputs the input electrocardiogram data and the generated electrocardiogram data. The output 12 pieces of electrocardiogram data may be output through the terminal of the subject to be measured, or may be output to a terminal used by a medical person. Then, the medical person diagnoses a heart disease by using the output electrocardiogram data.

As described above, according to the present invention, n pieces of additional electrocardiogram data are generated using electrocardiogram data, measured from one electrode or three electrodes, by means of a deep learning algorithm, thereby increasing the accuracy of diagnosing heart-related diseases. In addition, according to the present invention, it may be applied to portable wearable electrocardiogram devices, so that it can be used at home or in daily life.

Although the present invention has been described with reference to the embodiments shown in the drawings, this is merely illustrative. It will be understood by those of ordinary skill in the art to which the corresponding technology pertains that various modifications may be made therefrom. Therefore, the true technical protection range of the present invention should be defined by the scope of the following claims.

## Claims

1. An apparatus (100) for generating an electrocardiogram based on a generative adversarial network algorithm, the apparatus (100) comprising:
an input unit (110) configured to receive electrocardiogram data of a patient who wants his or her disease to be diagnosed;
a control unit (120) configured to generate a plurality of synthesized electrocardiogram data by inputting the received electrocardiogram data to a previously trained generative adversarial network algorithm;
an output unit (140) configured to output the received actual electrocardiogram data of the patient and the plurality of generated electrocardiogram data; and
a training unit (130) configured to extract lead electrocardiogram data from overall electrocardiogram data of a patient diagnosed with a heart disease and to train to generate a plurality of synthesized electrocardiogram data by inputting the extracted lead electrocardiogram data to a previously constructed generative adversarial network algorithm,
wherein the training unit (130) comprises:
a first generative model configured to generate n pieces of synthesized electrocardiogram data from the lead electrocardiogram data extracted from the input overall electrocardiogram data; and a second generative model configured to generate m pieces of synthesized electrocardiogram data from the n pieces of synthesized electrocardiogram data generated by the first generative model.

2. The apparatus (100) of claim 1, wherein the training unit (130) comprises:
a first discriminative model configured to receive the lead electrocardiogram data or m pieces of synthesized electrocardiogram data and to determine whether the data is actual data or has been synthesized; and
a second discriminative model configured to receive overall electrocardiogram data exclusive of the lead electrocardiogram data or n pieces of synthesized electrocardiogram data and to determine whether the data is actual data or has been synthesized.

3. A method of generating an electrocardiogram using an apparatus (100) for generating an electrocardiogram, the method comprising:
extracting (S320) lead electrocardiogram data from overall electrocardiogram data of a patient diagnosed with a heart disease and training to generate a plurality of synthesized electrocardiogram data by inputting the extracted lead electrocardiogram data to a previously constructed generative adversarial network algorithm;
receiving (S360) electrocardiogram data of a patient who wants his or her disease to be diagnosed;
generating (S370) a plurality of synthesized electrocardiogram data by inputting the received electrocardiogram data to a previously trained generative adversarial network algorithm; and
outputting (S380) the received actual electrocardiogram data of the patient and the plurality of generated electrocardiogram data,
wherein training (S320) to generate the plurality of synthesized electrocardiogram data comprises:
generating n pieces of synthesized electrocardiogram data from the lead electrocardiogram data extracted from the input overall electrocardiogram data by using a first generative model; and
generating m pieces of synthesized electrocardiogram data from the n pieces of synthesized electrocardiogram data generated by the first generative model by using a second generative model.

4. The method of claim 3, wherein training to generate the plurality of synthesized electrocardiogram data comprises:
receiving the lead electrocardiogram data or m pieces of synthesized electrocardiogram data and determining whether the data is actual data or has been synthesized by using a first discriminative model; and
receiving overall electrocardiogram data exclusive of the lead electrocardiogram data or n pieces of synthesized electrocardiogram data and determining whether the data is actual data or has been synthesized by using a second discriminative model.

## Patentansprüche

1. Vorrichtung (100) zum Generieren eines Elektrokardiogramms auf der Grundlage eines Algorithmus eines generativen adversariellen Netzwerks, wobei die Vorrichtung (100) umfasst:
eine Eingabeeinheit (110), die dazu eingerichtet ist, Elektrokardiogrammdaten eines Patienten zu empfangen, der seine Erkrankung diagnostiziert haben will;
eine Steuereinheit (120), die dazu eingerichtet ist, mehrere synthetisierte Elektrokardiogrammdaten zu generieren, indem sie die empfangenen Elektrokardiogrammdaten in einen zuvor trainierten Algorithmus eines generativen adversariellen Netzwerks eingibt;
eine Ausgabeeinheit (140), die dazu eingerichtet ist, die empfangenen tatsächlichen Elektrokardiogrammdaten des Patienten und die mehreren generierten Elektrokardiogrammdaten auszugeben; und
eine Trainingseinheit (130), die dazu eingerichtet ist, Ableitungs-Elektrokardiogrammdaten aus Gesamt-Elektrokardiogrammdaten eines Patienten, bei dem eine Herzerkrankung diagnostiziert wurde, zu extrahieren und zu trainieren, mehrere synthetisierte Elektrokardiogrammdaten zu generieren, indem die extrahierten Ableitungs-Elektrokardiogrammdaten in einen zuvor konstruierten Algorithmus eines generativen adversariellen Netzwerks eingegeben werden,
wobei die Trainingseinheit (130) umfasst:
ein erstes generatives Modell, das dazu eingerichtet ist, n synthetisierte Elektrokardiogrammdatenelemente aus den Ableitungs-Elektrokardiogrammdaten zu generieren, die aus den eingegebenen Gesamt-Elektrokardiogrammdaten extrahiert wurden; und ein zweites generatives Modell, das dazu eingerichtet ist, m synthetisierte Elektrokardiogrammdatenelemente aus den n synthetisierten Elektrokardiogrammdatenelementen zu generieren, die durch das erste generative Modell generiert wurden.

2. Vorrichtung (100) nach Anspruch 1, wobei die Trainingseinheit (130) umfasst:
ein erstes diskriminatives Modell, das dazu eingerichtet ist, die Ableitungs-Elektrokardiogrammdaten oder m synthetisierten Elektrokardiogrammdatenelemente zu empfangen und zu bestimmen, ob die Daten tatsächliche Daten sind oder synthetisiert wurden; und
ein zweites diskriminatives Modell, das dazu eingerichtet ist, die Gesamt-Elektrokardiogrammdaten ohne die Ableitungs-Elektrokardiogrammdaten oder n synthetisierten Elektrokardiogrammdaten zu empfangen und zu bestimmen, ob die Daten tatsächliche Daten sind oder synthetisiert wurden.

3. Verfahren zum Generieren eines Elektrokardiogramms unter Verwendung einer Vorrichtung (100) zum Generieren eines Elektrokardiogramms, wobei das Verfahren umfasst:
Extrahieren (S320) von Ableitungs-Elektrokardiogrammdaten aus den Gesamt-Elektrokardiogrammdaten eines Patienten, bei dem eine Herzerkrankung diagnostiziert wurde, und Trainieren, mehrere synthetisierte Elektrokardiogrammdaten zu generieren, indem die extrahierten Ableitungs-Elektrokardiogrammdaten in einen zuvor konstruierten Algorithmus eines generativen adversariellen Netzwerks eingegeben werden,
Empfangen (S360) von Elektrokardiogrammdaten eines Patienten, der seine Erkrankung diagnostiziert haben will;
Generieren (S370) mehrerer synthetisierter Elektrokardiogrammdaten, indem die empfangenen Elektrokardiogrammdaten in einen zuvor trainierten Algorithmus eines generativen adversariellen Netzwerks eingegeben werden; und
Ausgeben (S380) der empfangenen tatsächlichen Elektrokardiogrammdaten des Patienten und der mehreren generierten Elektrokardiogrammdaten,
wobei das Trainieren (S320) zum Generieren der mehreren synthetisierten Elektrokardiogrammdaten umfasst:
Generieren von n synthetisierten Elektrokardiogrammdaten aus den Ableitungs-Elektrokardiogrammdaten, die aus den eingegebenen Gesamt-Elektrokardiogrammdaten extrahiert wurden, unter Verwendung eines ersten generativen Modells; und
Generieren von m synthetisierten Elektrokardiogrammdaten aus den n synthetisierten Elektrokardiogrammdatenelementen, die durch das erste generative Modell generiert wurden, unter Verwendung eines zweiten generativen Modells.

4. Verfahren nach Anspruch 3, wobei das Trainieren zum Generieren der mehreren synthetisierten Elektrokardiogrammdaten umfasst:
Empfangen der Ableitungs-Elektrokardiogrammdaten oder m synthetisierten Elektrokardiogrammdatenelemente und Bestimmen, ob die Daten tatsächliche Daten sind oder synthetisiert wurden, unter Verwendung eines ersten diskriminativen Modells; und
Empfangen von Gesamt-Elektrokardiogrammdaten ohne die Ableitungs-Elektrokardiogrammdaten oder n synthetisierten Elektrokardiogrammdaten und Bestimmen, ob die Daten tatsächliche Daten sind oder synthetisiert wurden, unter Verwendung eines zweiten diskriminativen Modells.

## Revendications

1. Dispositif (100) pour la génération d'un électrocardiogramme sur la base d'un algorithme de réseau antagoniste génératif, le dispositif (100) comprenant :
une unité d'entrée (110) configurée pour recevoir des données d'électrocardiogramme d'un patient souhaitant faire diagnostiquer sa maladie ;
une unité de commande (120) configurée pour générer une pluralité de données d'électrocardiogramme synthétisées par entrée des données d'électrocardiogramme reçues dans un algorithme de réseau antagoniste génératif préalablement formé ;
une unité de sortie (140) configurée pour délivrer en sortie les données d'électrocardiogramme réelles reçues du patient et la pluralité de données d'électrocardiogramme générées ; et
une unité de formation (130) configurée pour extraire des données d'électrocardiogramme de dérivation à partir de données d'électrocardiogramme globales d'un patient chez lequel une maladie cardiaque a été diagnostiquée et pour effectuer une formation en vue de générer une pluralité de données d'électrocardiogramme synthétisées par entrée des données d'électrocardiogramme de dérivation extraites dans un algorithme de réseau antagoniste génératif préalablement construit,
l'unité de formation (130) comprenant :
un premier modèle génératif configuré pour générer n données d'électrocardiogramme synthétisées à partir des données d'électrocardiogramme de dérivation extraites des données d'électrocardiogramme globales d'entrée ; et un deuxième modèle génératif configuré pour générer m données d'électrocardiogramme synthétisées à partir des n données d'électrocardiogramme synthétisées générées par le premier modèle génératif.

2. Dispositif (100) selon la revendication 1, l'unité de formation (130) comprenant :
un premier modèle discriminatif configuré pour recevoir les données d'électrocardiogramme de dérivation ou m données d'électrocardiogramme synthétisées et pour déterminer si les données sont des données réelles ou ont été synthétisées ; et
un deuxième modèle discriminatif configuré pour recevoir des données d'électrocardiogramme globales à l'exclusion des données d'électrocardiogramme de dérivation ou n données d'électrocardiogramme synthétisées et pour déterminer si les données sont des données réelles ou ont été synthétisées.

3. Procédé de génération d'un électrocardiogramme à l'aide d'un dispositif (100) pour la génération d'un électrocardiogramme, le procédé comprenant :
l'extraction (S320) de données d'électrocardiogramme de dérivation à partir de données d'électrocardiogramme globales d'un patient chez lequel une maladie cardiaque a été diagnostiquée et la formation en vue de générer une pluralité de données d'électrocardiogramme synthétisées par entrée des données d'électrocardiogramme de dérivation extraites dans un algorithme de réseau antagoniste génératif préalablement construit ;
la réception (S360) de données d'électrocardiogramme d'un patient souhaitant faire diagnostiquer sa maladie ;
la génération (S370) d'une pluralité de données d'électrocardiogramme synthétisées par entrée des données d'électrocardiogramme reçues dans un algorithme de réseau antagoniste génératif préalablement formé ; et
la délivrance en sortie (S380) des données d'électrocardiogramme réelles reçues du patient et de la pluralité de données d'électrocardiogramme générées,
la formation (S320) en vue de générer la pluralité de données d'électrocardiogramme synthétisées comprenant :
la génération de n données d'électrocardiogramme synthétisées à partir des données d'électrocardiogramme de dérivation extraites des données d'électrocardiogramme globales d'entrée à l'aide d'un premier modèle génératif ; et
la génération de m données d'électrocardiogramme synthétisées à partir des n données d'électrocardiogramme synthétisées générées par le premier modèle génératif à l'aide d'un deuxième modèle génératif.

4. Procédé selon la revendication 3, la formation en vue de générer la pluralité de données d'électrocardiogramme synthétisées comprenant :
la réception des données d'électrocardiogramme de dérivation ou de m données d'électrocardiogramme synthétisées et la détermination de si les données sont des données réelles ou ont été synthétisées à l'aide d'un premier modèle discriminatif ; et
la réception de données d'électrocardiogramme globales à l'exclusion des données d'électrocardiogramme de dérivation ou de n données d'électrocardiogramme synthétisées et la détermination de si les données sont des données réelles ou ont été synthétisées à l'aide d'un deuxième modèle discriminatif.
